# DEMANDE DE BREVET EUROPEEN

(11) **EP 1 900 723 A1**
(43) Date de publication de la demande: **19.03.2008**
(21) Numéro de dépôt: 07290820.5
(22) Date de dépôt: 29.06.2007
(51) Int. Cl.: C07C 233/18, C07C 233/20, C07C 233/60, C07C 233/78, C07C 247/10, C07C 275/24, C07C 309/66, C07C 311/05, C07D 295/12

(54) **Neuveaux dérivés naphtaléniques, leur procédé de préparation et les compositions pharmaceutiques qui les contiennent**

(30) Priorité: 30.06.2006 FR 0605916
(71) Demandeur: Les Laboratoires Servier, 92415 Courbevoie Cedex (FR)
(72) Inventeur: Yous, Said, c/o Résidence Marthe Alexandre, 59120 Loos (FR); Peres, Basile, 09000 Foix (FR); Sabaouni, Ahmed, 59280 Armentieres (FR); Berthelot, Pascal, 59320 Haubourdin (FR); Spedding, Michael, 78110 Le Vesinet (FR); Delagrange, Philippe, 92130 Issy Les Molineaux (FR); Caignard, Daniel-Henri, 95000 Lille (FR); Desroses, Matthieu, 59800 Lille (FR); Boutin, Jean-Albert, 92150 Suresnes (FR); Audinot, Valérie, 78300 Poissy (FR)

(57) **Abrégé**

Composés de formule (I) : 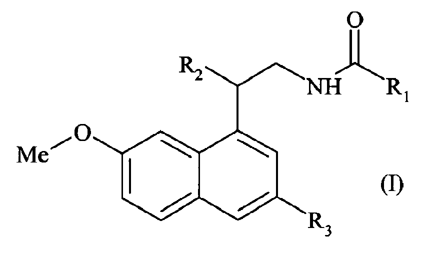 dans laquelle :
◆ R₁ représente un groupement R₄ ou NHR₄, dans lesquels R₄ est tel que défini dans la description ;
◆ R₂ représente un groupement alkyle (C₁-C₆) linéaire ou ramifié substitué ;
◆ R₃ représente un atome d'hydrogène ou d'halogène, ou un groupement alkyle (C₁-C₆) linéaire ou ramifié ou alkényle (C₁-C₆) linéaire ou ramifié.

Médicaments.

## Description

La présente invention concerne de nouveaux dérivés naphtaléniques, leur procédé de préparation et les compositions pharmaceutiques qui les contiennent.

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle des rythmes circadiens. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), analgésiques (Pharmacopsychiat., 1987, 20, pp. 222-223), ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands sélectifs.

De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sites de liaisons mélatoninergiques.

Ils présentent par ailleurs une forte affinité pour le récepteur 5-HT_{2C}, ce qui a pour effet de renforcer les propriétés observées avec les récepteurs mélatoninergiques, notamment dans le domaine de la dépression.

La présente invention concerne plus particulièrement les composés de formule (I) : 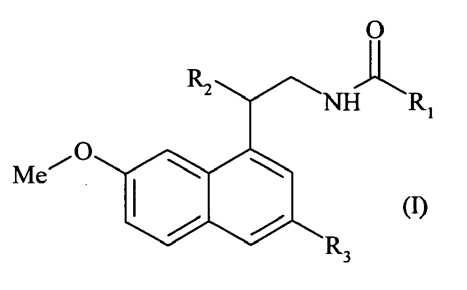 dans laquelle :
◆ R₁ représente un groupement R₄ ou NHR₄, dans lesquels R₄ représente groupement alkyle (C₁-C₆) linéaire ou ramifié, alkényle (C₁-C₆) linéaire ou ramifié, halogénoalkyle (C₁-C₆) linéaire ou ramifié, polyhalogénoalkyle (C₁-C₆) linéaire ou ramifié, cycloalkyle (C₃-C₈), cycloalkyl(C₃-C₈)alkyle (C₁-C₆) dont la partie alkyle peut être linéaire ou ramifiée, aryle, arylalkyle (C₁-C₆) dont la partie alkyle peut être linéaire ou ramifiée, hétéroaryle ou hétéroarylalkyle (C₁-C₆) dont la partie alkyle peut être linéaire ou ramifiée,
◆ R₂ représente un groupement alkyle (C₁-C₆) linéaire ou ramifié substitué par un groupement alkoxy (C₁-C₆) linéaire ou ramifié, OH, OSO₂Me, N₃, NRR', NHCOR" ou par un groupement NHSO₂R",
   dans lesquels R et R', identiques ou différents, représentent chacun un atome d'hydrogène ou un groupement alkyle (C₁-C₆) linéaire ou ramifié, cycloalkyle (C₃-C₈), aryle ou arylalkyle (C₁-C₆) dont la partie alkyle peut être linéaire ou ramifiée, ou R et R' forment ensemble avec l'atome d'azote qui les porte un cycle comportant 5 ou 6 chaînons et pouvant contenir un autre hétéroatome choisi parmi azote, oxygène et soufre,
   et R" représente un groupement alkyle (C₁-C₆) linéaire ou ramifié, cycloalkyle (C₃-C₈), aryle ou arylalkyle (C₁-C₆) dont la partie alkyle peut être linéaire ou ramifiée,
◆ R₃ représente un atome d'hydrogène ou d'halogène, ou un groupement alkyle (C₁-C₆) linéaire ou ramifié ou alkényle (C₁-C₆) linéaire ou ramifié,
   étant entendu que :
   - lorsque R₁ représente un groupement méthyle et R₂ représente un groupement hydroxyméthyle, alors R₃ ne peut représenter un atome d'hydrogène,
   - par "aryle", on entend un groupement phényle, naphtyle ou biphényle,
   - par "hétéroaryle", on entend tout groupement aromatique mono ou bicyclique contenant 1 à 3 hétéroatomes choisis parmi oxygène, soufre et azote,
les groupements aryle et hétéroaryle ainsi définis pouvant être substitués par 1 à 3 groupements choisis parmi alkyle (C₁-C₆) linéaire ou ramifié, alkoxy (C₁-C₆) linéaire ou ramifié, hydroxy, carboxy, formyle, nitro, cyano, haloalkyle (C₁-C₆) linéaire ou ramifié, polyhalogénoalkyle (C₁-C₆) linéaire ou ramifié, alkyloxycarbonyle, ou atomes d'halogène,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc.
Les composés préférés de l'invention sont les composés de formule (I) pour lesquels R₁ représente un groupement alkyle (C₁-C₆) linéaire ou ramifié comme par exemple les groupements méthyle ou éthyle.

R₂ représente avantageusement un groupement alkyle substitué par un groupement OH ou alkoxy.

R₃ représente avantageusement un atome d'hydrogène.

Encore plus particulièrement, l'invention concerne les composés qui sont le *N*-[3-méthoxy-2-(7-méthoxy-1-naphtyl)propyl]acétamide, le *N*-[4-hydroxy-2-(7-méthoxy-1-naphtyl) butyl]propanamide et le *N*-[4-hydroxy-2-(7-méthoxy-1-naphtyl) butyl]acétamide.

Les énantiomères, diastéréoisomères ainsi que les sels d'addition à un acide ou à une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (II) : 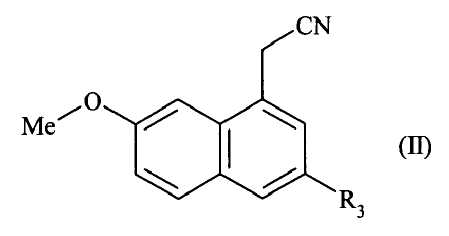 dans laquelle R₃ est tel que défini dans la formule (I),
que l'on soumet en milieu basique à l'action du carbonate de diméthyle pour conduire au composé de formule (III) : 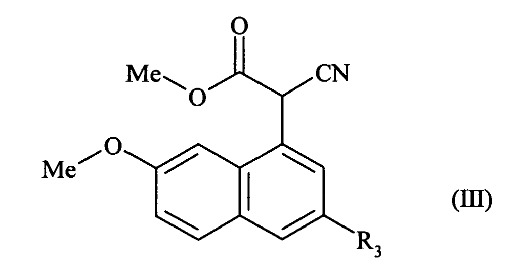 dans laquelle R₃ est tel que défini précédemment, sur lequel on condense éventuellement un composé de formule Hal-(CH₂)ₙ-COOMe dans laquelle Hal représente un atome d'halogène et n vaut 1 à 6 pour conduire au composé de formule (IV) : 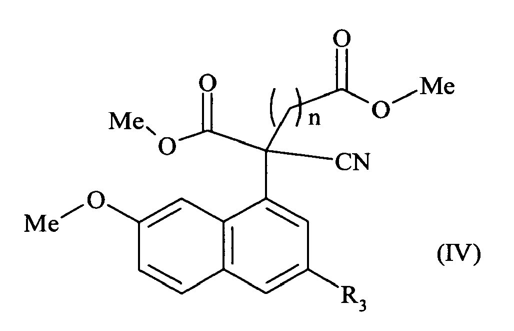 dans laquelle R₃ et n sont tels que définis précédemment,
que l'on soumet à l'action du bromure de lithium pour conduire au composé de formule (V) : 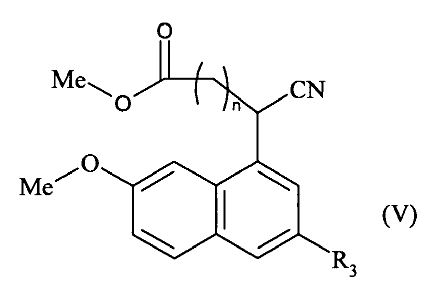 dans laquelle R₃ et n sont tels que définis précédemment, l'ensemble des composés de formule (III) et (V) formant le composé de formule (VI) : 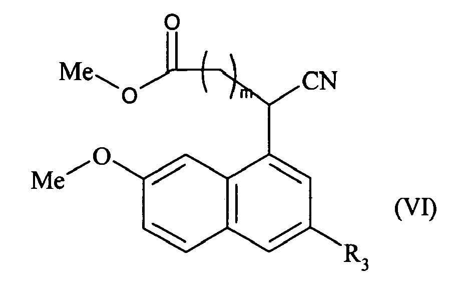 dans laquelle R₃ est tel que défini précédemment et m vaut 0, 1, 2, 3, 4, 5 ou 6,
que l'on soumet à une réduction en présence d'un hydrure pour conduire au composé de formule (VII) : 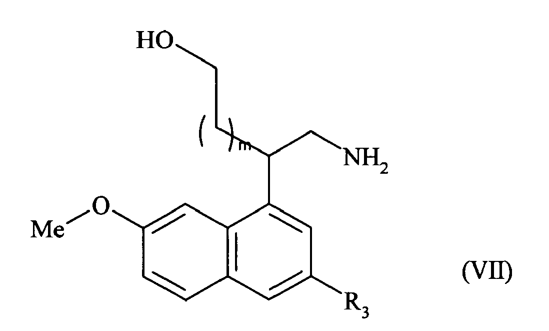 dans laquelle R₃ et m sont tels que définis précédemment,
sur lequel on condense un composé de formule R₁C(O)Cl pour conduire au composé de formule (I/a), cas particulier des composés de formule (1) : 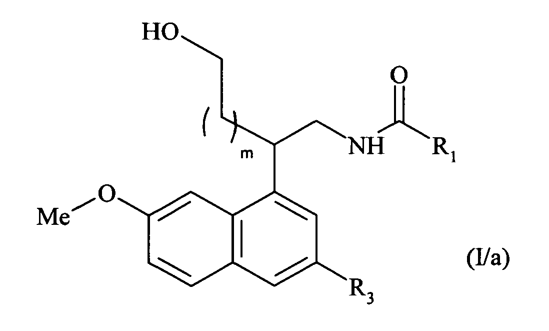 dans laquelle R₃, m et R₁ sont tels que définis précédemment, qui est éventuellement :
- soit soumis à l'action d'un halogénure d'alkyle en milieu basique pour conduire au composé de formule (I/b), cas particulier des composés de formule (1) : 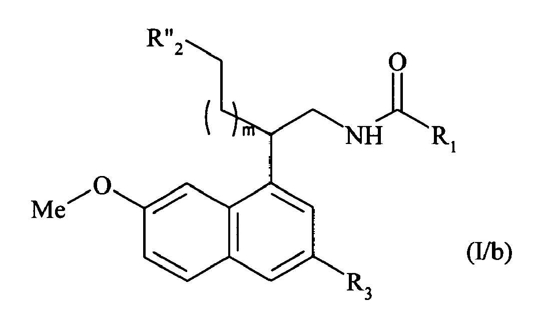 dans laquelle R₃, m et R₁ sont tels que définis précédemment et R"₂ représente un groupement alkoxy (C₁-C₆) linéaire ou ramifié,
- soit sur lequel on condense le chlorure de mésylate en milieu basique pour conduire au composé de formule (I/c), cas particulier des composés de formule (1) : 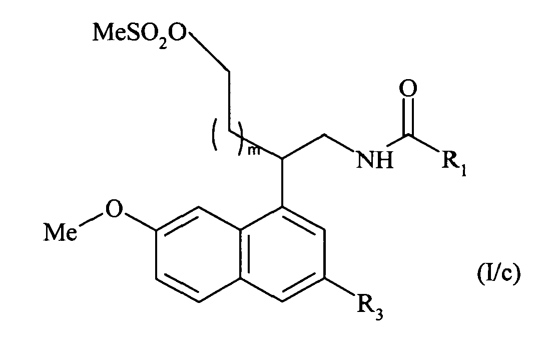 dans laquelle R₃, m et R₁ sont tels que définis précédemment, sur lequel on condense éventuellement :
   □ soit une amine de formule HNRR' dans laquelle R et R' sont tels que définis dans la formule (I) pour conduire au composé de formule (I/d), cas particulier des composés de formule (1) : 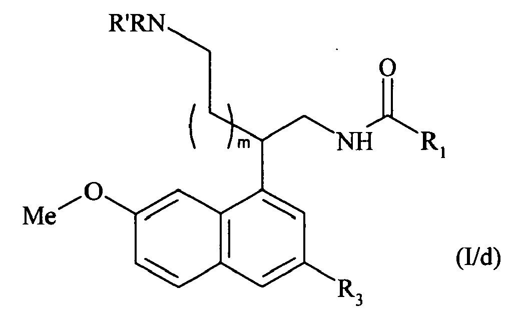 dans laquelle R₃, m, R, R' et R₁ sont tels que définis précédemment,
   □ soit un azidure pour conduire au composé de formule (I/e), cas particulier des composés de formule (1), 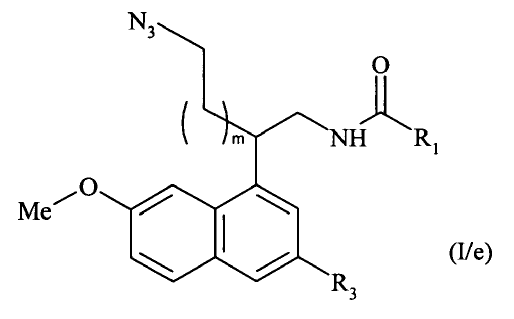 dans laquelle R₃, m et R₁ sont tels que définis précédemment,
      que l'on soumet éventuellement à une réduction en présence de charbon palladié, suivi éventuellement de la mono ou bis condensation d'un composé de formule R-Hal dans laquelle R est tel que défini dans la formule (I), pour conduire au composé de formule (I/d) tel que défini précédemment pour lequel R et R' ne forment pas ensemble avec l'atome d'azote qui les porte un groupement cyclique,
composé de formule (I/d) qui, lorsque R et R' représentent simultanément un atome d'hydrogène est éventuellement soumis à l'action d'un composé de formule R"C(O)Cl ou R"SO₂Cl dans lesquelles R" est tel que défini précédemment, pour conduire au composé de formule (I/f), cas particulier des composés de formule (1) : 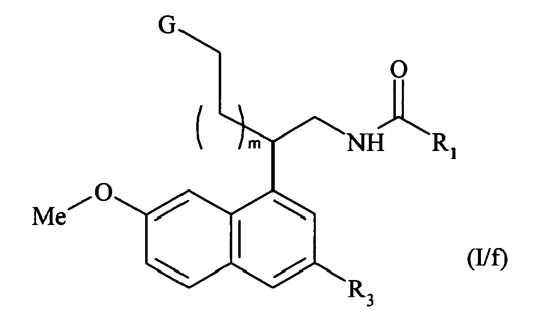 dans laquelle R₃, m et R₁ sont tels que définis précédemment, et G représente un groupement NHCOR" ou NHSO₂R", dans lesquels R" est tel que défini dans la formule (I),
les composés de formule (I/a) à (I/f) formant l'ensemble des composés de formule (I) qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

Les composés de formule (II) sont soit commerciaux, soit accessibles à l'homme du métier par des réactions chimiques classiques et décrites dans la littérature.

L'étude pharmacologique des dérivés de l'invention a montré qu'ils étaient atoxiques, doués d'une forte affinité sélective pour les récepteurs de la mélatonine et possédaient d'importantes activités sur le système nerveux central et, en particulier, on a relevé des propriétés thérapeutiques sur les troubles du sommeil, des propriétés antidépressives, anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières ou de la dépression majeure, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

Par exemple, les composés seront utilisés dans le traitement de la dépression majeure, des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon.

### Exemple 1 : N-[3-Hydroxy-2-(7-méthoxy-1-naphtyl)propyl]cyclopropane carboxamide

### Stade A : Cyano(7-méthoxy-1-naphtyl)acétate de méthyle

Le (7-méthoxy-napht-1-yl)acétonitrile (20 g) est dissous dans 150 ml de tétrahydrofurane anhydre. L'hydrure de sodium (202,8 mmol) est ajouté à température ambiante, et le milieu est chauffé à reflux pendant 30 minutes. Le carbonate de diméthyle (12 ml) est ajouté avec précaution et le milieu réactionnel est chauffé à reflux pendant 30 minutes. Le milieu est versé dans l'eau glacée et la phase aqueuse est acidifiée avec 21 ml d'une solution d'acide chlorhydrique à 37 % puis extraite par 2 fois 100 ml d'éther. La phase organique est lavée à l'eau, séchée, décolorée et évaporée. L'huile obtenue précipite dans l'éther et le précipité formé est essoré puis recristallisé pour conduire au produit du titre sous la forme d'un solide blanc.
*Point de fusion : 80-82°C*

### Stade B : 3-Amino-2-(7-méthoxy-1-naphtyl)-1-propanol, chlorhydrate

Le chlorure d'aluminium (80 mmol), solubilisé dans 200 ml d'éther anhydre, est ajouté à une suspension d'hydrure mixte d'aluminium et de lithium à 0°C dans 300 ml d'éther anhydre. Après 10 minutes d'agitation le composé obtenu au Stade A (20 mmol) est ajouté en solution dans 200 ml d'éther anhydre. Après 30 minutes, le milieu est hydrolysé à froid et avec précaution avec une solution de soude (10 g ; 40 ml). Le précipité formé est ensuite filtré et abondamment lavé à l'éther. Le résidu obtenu après évaporation est repris par l'eau et la phase aqueuse est extraite au dichlorométhane. La phase organique est ensuite lavée à l'eau, séchée, décolorée, puis traitée par l'acide chlorhydrique gazeux et évaporée. L'huile obtenue précipite dans l'acétate d'éthyle et le précipité formé est essoré puis recristallisé pour conduire au produit du titre sous la forme d'un solide blanc.
*Point de fusion : 164-166°C*

### Stade C : N-[3-Hydroxy-2-(7-méthoxy-1-naphtyl)propyl]cyclopropane carboxamide

Le composé obtenu au Stade B (3,73 mmol) est mis en solution dans 100 ml d'un mélange eau-acétate d'éthyle (50/50). Le carbonate de potassium (11,2 mmol) est ajouté et le milieu réactionnel est refroidi à 0°C avec un bain de glace. Le chlorure de cyclopropanoyle (4,4 mmol) est ajouté goutte à goutte et le milieu est agité pendant 15 minutes à froid. En fin de réaction, la phase organique est lavée avec une solution d'acide chlorhydrique (1M), lavée à l'eau, séchée et évaporée sous pression réduite. Le solide obtenu est recristallisé dans du toluène pour conduire au produit du titre sous la forme d'un solide blanc.
*Point de fusion : 153-154°C*

| *Microanalyse élémentaire :* | | | |
|---|---|---|---|
| *%* | *C* | *H* | *N* |
| *Calculé :* | *72,22* | *7,07* | *4,68* |
| *Trouvé :* | *72,38* | *7,28* | *4,53* |

### Exemple 2 : N-[3-Hydroxy-2-(7-méthoxy-1-naphtyl)propyl]acrylamide

On procède comme dans le Stade C de l'Exemple 1 en remplaçant le chlorure de cyclopropanoyle par le chlorure d'acryloyle.
*Point de fusion : 150-152°C*

| *Microanalyse élémentaire :* | | | |
|---|---|---|---|
| % | *C* | *H* | *N* |
| *Calculé :* | *71,56* | *6,71* | *4,91* |
| *Trouvé :* | *71,37* | *6, 81* | *4, 82* |

### Exemple 3 : N-[3-Hydroxy-2-(7-méthoxy-1-naphtyl)propyl]-3-butènamide

L'acide vinylacétique (5 mmol) est mis en solution dans 40 ml de dichlorométhane et la solution est refroidie à 0°C. L'EDCI (6 mmol) est ajouté par petites portions et le milieu est agité à 0°C pendant 30 minutes. Le composé obtenu au Stade B de l'Exemple 1 sous forme base, en solution dans 20 ml de dichlorométhane, est ajouté au milieu. Après 30 minutes d'agitation à 0°C le milieu réactionnel est versé dans l'eau. La phase organique est lavée avec une solution d'acide chlorhydrique (1M), puis avec une solution d'hydogénocarbonate de sodium (1M) et à l'eau. La phase organique est ensuite séchée et évaporée. L'huile obtenue précipite dans l'éther et le solide obtenu est essoré puis recristallisé dans du toluène pour conduire au produit du titre sous la forme d'un solide blanc.
*Point de fusion : 86-88°C*

### Exemple 4 : 2,2,2-Trifluoro-N-[3-hydroxy-2-(7-méthoxy-1-naphtyl)propyl]acétamide

Le composé obtenu au Stade B de l'Exemple 1 (5,6 mmol) est mis en solution dans 40 ml de tétrahydrofurane en présence de triéthylamine (11,2 mmol) et l'anhydride trifluoroacétique (5 mmol) est ajouté. Le milieu est agité à température ambiante pendant 10 minutes, concentré sous pression réduite puis versé dans l'eau. La phase aqueuse est extraite par 2 fois 60 ml d'éther, la phase organique est lavée avec une solution d'acide chlorhydrique (1M), puis lavée à l'eau, séchée et évaporée. L'huile obtenue précipite dans un mélange éther-éther de pétrole (50/50), le précipité formé est essoré puis recristallisé dans du toluène pour conduire au composé du titre sous la forme d'un solide blanc.
*Point de fusion : 138-140°C*

### Exemple 5 : 4-Chloro-N-[3-hydroxy-2-(7-méthoxy-1-naphtyl)propyl]butanamide

On procède comme dans le Stade C de l'Exemple 1 en remplaçant le chlorure de cyclopropanoyle par le chlorure de 4-chlorobutanoyle.
*Huile blanchâtre*

### Exemple 6 : N-[3-Méthoxy-2-(7-méthoxy-1-naphtyl)propyl]acétamide

### Stade A : 3-Méthoxy-2-(7-méthoxy-1-naphtyl)-1-propanamine, chlorhydrate

Le produit du titre est obtenu par alkylation du composé obtenu au Stade B de l'Exemple 1.

### Stade B : N-[3-Méthoxy-2-(7-méthoxy-1-naphtyl)propyl]acétamide

On procède comme dans le Stade C de l'Exemple 1 à partir du composé obtenu au Stade A et en remplaçant le chlorure de cyclopropanoyle par le chlorure d'acétyle.
*Point de fusion : 82-84°C*

| *Microanalyse élémentaire :* | | | |
|---|---|---|---|
| % | *C* | *H* | *N* |
| *Calculé :* | *71,05* | *7,36* | *4,87* |
| *Trouvé :* | *70,77* | *7,57* | *4,78* |

### Exemple 7 : N-[4-Hydroxy-2-(7-méthoxy-1-naphtyl)butyl]acétamide

### Stade A : 2-Cyano-2-(7-méthoxy-1-naphtyl)succinate de diméthyle

Le composé obtenu au Stade A de l'Exemple 1 (19,6 mmol) est mis en solution dans 80 ml d'acétone en présence de bromure de tétrabutylamonium (200 mg) et de carbonate de potassium (58,8 mmol). Le milieu est chauffé à reflux pendant 20 minutes et le bromoacétate de méthyle (23,5 mmol) est ajouté goutte à goutte. Le milieu est maintenu à reflux pendant 10 minutes et filtré en fin de réaction. Le carbonate de potassium est lavé à l'acétone et le filtrat est évaporé. Le solide obtenu est essoré dans l'éther puis recristallisé dans un mélange toluène/cyclohexane (3/2) pour conduire au produit du titre sous la forme d'un solide beige.
*Point de fusion : 119-121°C*

### Stade B : 3-Cyano-3-(7-méthoxy-1-naphtyl)propanoate de méthyle

Le composé obtenu dans le Stade A (16 mmol) est mis en solution dans 15 ml de diméthylformamide puis le bromure de lithium (16 mmol) et l'eau (16 mmol) sont ajoutés à la solution. Le milieu réactionnel est ensuite chauffé à reflux pendant 16 heures et versé dans 30 ml d'eau en fin de réaction. Le précipité formé est séché puis recristallisé dans un mélange toluène/cyclohexane (1/3) pour conduire au produit du titre sous la forme d'un solide beige.
*Point de fusion : 113-114°C*

### Stade C : 4-(Acétylamino)-3-(7-méthoxy-1-naphtyl)butanoate de méthyle

Le composé obtenu au Stade B (18,5 mmol) est mis en solution dans 200 ml d'anhydride acétique et le nickel de Raney (2 g) est ajouté à la solution. Le milieu réactionnel est placé sous une pression de 30 bars d' hydrogène et chauffé à 60°C pendant 3 heures, puis filtré et évaporé à sec. Le résidu obtenu est repris par 100 ml d'eau et la phase aqueuse est extraite par 2 fois 100 ml d'éther. La phase organique est lavée avec une solution d'hydrogénocarbonate de sodium (1M), lavée à l'eau, séchée, et évaporée. L'huile obtenue précipite dans l'éther et le précipité formé est essoré puis recristallisé dans l'éther diisopropylique pour conduire au produit du titre sous la forme d'un solide blanc.
*Point de fusion : 94-95°C*

### Stade D : N-[4-Hydroxy-2-(7-méthoxy-1-naphtyl)butyl]acétamide

Le composé obtenu au Stade C (6,3 mmol) est mis en solution dans 200 ml d'éther anhydre et l'hydrure mixte d'aluminium et de lithium (9,45 mmol) est ajouté par petites portions. Le milieu est agité à température ambiante pendant 6 heures et neutralisé avec 2 ml d'eau. La phase éthérée est lavée à l'eau, séchée et évaporée. L'huile obtenue est purifiée sur gel de silice avec comme éluant un mélange acétone-acétate d'éthyle (40/60) pour conduire au produit du titre sous la forme d'un solide blanc.
*Point de fusion : 143-145°C*

### Exemple 8 : 3-(Acétylamino)-2-(7-méthoxy-1-naphtyl)propyl méthanesulfonate

### Stade A : 3-Amino-2-(7-méthoxy-1-naphtyl)propyl méthanesulfonate, chlorhydrate

Le produit du titre est obtenu par mésylation du composé obtenu au Stade B de l'Exemple 1.

### Stade B : 3-(Acétylamino)-2-(7-méthoxy-1-naphtyl)propyl méthanesulfonate

On procède comme dans le Stade C de l'Exemple 1 à partir du composé obtenu au Stade A et en remplaçant le chlorure de cyclopropanoyle par le chlorure d'acétyle. Le composé du titre est obtenu sous la forme d'un solide blanc.
*Point de fusion : 104-106°C*

### Exemple 9 : 2-(7-Méthoxy-1-naphtyl)-3-(propionylamino)propyl méthanesulfonate

On procède comme dans l'Exemple 8 en remplaçant au Stade B le chlorure d'acétyle par le chlorure de propanoyle. Le composé du titre est obtenu sous la forme d'un solide blanc.
*Point de fusion : 118-120°C*

### Exemple 10 : N-[2-(7-Méthoxy-1-naphtyl)-3-(4-morpholinyl)propyl]propanamide, chlorhydrate

Le composé obtenu dans l'Exemple 8 (4,26 mmol) et la morpholine (42,3 mmol) sont solubilisés dans 40 ml de tétrahydrofurane anhydre et le milieu réactionnel est chauffé à reflux sous courant d'argon pendant 24 heures. En fin de réaction le milieu est concentré sous vide puis versé dans l'eau. La phase aqueuse est extraite par 2 fois 50 ml d'éther et la phase organique est lavée à l'eau, puis lavée avec une solution d'acide chlorhydrique (1M). La phase aqueuse est ensuite alcalinisée par une solution de soude 15 % puis extraite par 2 fois 50 ml d'éther. La phase organique est lavée à l'eau, séchée, décolorée, puis traitée par l'éther saturé d'acide chlorhydrique. Le précipité formé est essoré puis recristallisé dans l'acétonitrile pour conduire au produit du titre sous la forme d'un solide blanc.
*Point de fusion : 125-126°C*

| *Microanalyse élémentaire :* | | | |
|---|---|---|---|
| % | *C* | *H* | *N* |
| *Calculé :* | *63,40* | *7,18* | *7,39* |
| *Trouvé :* | *63,38* | *7, 22* | *7,3 7* |

### Exemple 11 : N-[3-azido-2-(7-methoxy-1-naphthyl)propyl]acetamide

L'azoture de sodium (25,6 mmol) est mis en suspension dans 10 ml de diméthylformamide, le bromure de tétrabutylamonium (une pointe de spatule) est ajouté et le milieu est chauffé à 70°C. Le composé obtenu dans l'Exemple 8 (8,53 mmol) est ensuite ajouté en solution dans 20 ml de diméthylformamide et le milieu est agité à 70°C pendant deux heures. En fin de réaction, 40 ml d'eau sont ajoutés et la phase aqueuse est extraite par 3 fois 60 ml d'éther. La phase organique est ensuite lavée avec une solution d'acide chlorhydrique (2M) puis à l'eau. Après avoir été séchée la phase organique est évaporée pour conduire au produit du titre sous la forme d'une huile orangée.
*Huile orangée*

### Exemple 12 : N-[3-Azido-2-(7-méthoxy-1-naphtyl)propyl]propanamide

On procède comme dans l'Exemple 11 à partir du composé obtenu dans l'Exemple 9.
*Huile blanchâtre*

### Exemple 13 : N-[3-Amino-2-(7-méthoxy-1-naphtyl)propyl]acétamide, chlorhydrate

Le composé obtenu dans l'Exemple 11 (6,48 mmol) est solubilisé dans 50 ml de méthanol et le charbon palladié (200 mg) est ajouté à la solution. Le milieu est ensuite placé sous pression atmosphérique d'hydrogène et agité à température ambiante pendant 2 heures. En fin de réaction, le catalyseur est filtré et le méthanol est évaporé. Le résidu obtenu est repris par l'éther et l'insoluble formé est filtré. Le filtrat est ensuite traité par l'éther saturé d'acide chlorhydrique et le chlorhydrate formé est essoré puis recristallisé dans un mélange acétonitrile/méthanol (8/2) pour conduire au produit du titre sous la forme d'un solide blanc.
*Point de fusion : 230-231°C*

### Exemple 14 : N-13-Amino-2-(7-méthoxy-1-naphtyl)propyl]propanamide, chlorhydrate

On procède comme dans l'Exemple 13 à partir du composé obtenu dans l'Exemple 12.
*Point de fusion : 198-200°C*

### Exemple 15 : N-[3-(Acétylamino)-2-(7-méthoxy-1-naphtyl)propyl]acétamide

On procède comme dans le Stade C de l'Exemple 1 à partir du composé obtenu dans l'Exemple 13 et en remplaçant le chorure de cyclopropanoyle par le chlorure d'acétyle. Le produit du titre est recristallisé dans l'acétonitrile et obtenu sous la forme d'un solide blanc.
*Point de fusion : 199-200°C*

| *Microanalyse élémentaire :* | | | |
|---|---|---|---|
| *%* | *C* | *H* | *N* |
| *Calculé :* | *68,77* | *7,05* | *8,91* |
| *Trouvé :* | *68,67* | *7,15* | *8,89* |

### Exemple 16 : N-[3-(Acétylamino)-2-(7-méthoxy-1-naphtyl)propyl]propanamide

On procède comme dans l'Exemple 15 à partir du composé obtenu dans l'Exemple 13 en remplaçant le chlorure d'acétyle par le chlorure de propanoyle. Le produit du titre est recristallisé dans l'acétonitrile et obtenu sous la forme d'un solide blanc.
*Point de fusion : 148-150°C*

| *Microanalyse élémentaire :* | | | |
|---|---|---|---|
| *%* | *C* | *H* | *N* |
| *Calculé :* | *69,49* | *7,37* | *8,53* |
| *Trouvé :* | *69,74* | *7,46* | *8,42* |

### Exemple 17 : N-[3-(Acétylamino)-2-(7-méthoxy-1-naphtyl)propyl]butanamide

On procède comme dans l'Exemple 15 à partir du composé obtenu dans l'Exemple 13 en remplaçant le chlorure d'acétyle par le chlorure de butanoyle. Le produit du titre est recristallisé dans l'acétonitrile et obtenu sous la forme d'un solide blanc.
*Point de fusion : 150-152°C*

| *Microanalyse élémentaire :* | | | |
|---|---|---|---|
| *%* | *C* | *H* | *N* |
| *Calculé :* | *70,15* | *7,65* | *8,18* |
| *Trouvé :* | *70,22* | *7,33* | *8,25* |

### Exemple 18 : N-[3-(Acétylamino)-2-(7-méthoxy-1-naphtyl)propyl]cyclopropane carboxamide

On procède comme dans le Stade C de l'Exemple 1 à partir du composé obtenu dans l'Exemple 13. Le produit du titre est recristallisé dans l'acétonitrile et obtenu sous la forme d'un solide blanc.
*Point de fusion : 175-176°C*

| *Microanalyse élémentaire :* | | | |
|---|---|---|---|
| % | *C* | *H* | *N* |
| *Calculé :* | *70,57* | *7,11* | *8,23* |
| *Trouvé :* | *70,48* | *7,32* | *8,51* |

### Exemple 19 : N-[3-[Benzyl(méthyl)amino]-2-(7-méthoxy-1-naphtyl)propyl]acétamide

Le composé obtenu dans l'Exemple 8 (10 mmol) et la N-méthylbenzylamine (30 mmol) sont solubilisés dans 60 ml de tétrahydrofurane anhydre. Le milieu réactionnel est placé sous courant d'argon et chauffé à reflux durant 24 heures. En fin de réaction le milieu est concentré sous vide puis versé dans l'eau. La phase aqueuse est extraite par 2 fois 50 ml d'éther et la phase organique est lavée avec une solution d'acide chlorhydrique (2M). La phase aqueuse est ensuite alcalinisée avec une solution de soude (2M) puis extraite par 2 fois 50 ml d'éther. La phase éthérée est lavée à l'eau, séchée et évaporée sous pression réduite. L'huile obtenue est purifiée sur gel de silice avec comme éluant un mélange acétone-cyclohexane (50/50). Le solide obtenu après évaporation des fractions pures est essoré dans un mélange éther-éther de pétrole puis recristallisé dans l'éther diisopropylique.
*Point de fusion : 100-102°C*

### Exemple 20 : N-[2-(7-Méthoxy-1-naphtyl)-3-(méthylamino)propyl]acétamide

Le composé obtenu dans l'Exemple 19 (2,6 mmol) est solubilisé dans 40 ml de méthanol et le charbon palladié (une pointe de spatule) est ajouté à la solution. Le milieu réactionnel est agité à température ambiante sous pression atmosphérique d'hydrogène durant 24 heures. En fin de réaction le milieu est filtré puis concentré sous pression réduite et versé dans l'eau. La phase aqueuse est ensuite extraite par 2 fois 40 ml d'éther et la phase organique est lavée à l'eau, séchée puis traitée par l'éther saturé d'acide chlorhydrique. Le précipité formé est essoré dans l'éther et recristallisé dans l'acétone pour conduire au produit du titre sous la forme d'un solide blanc.
*Point de fusion : 126-128°C*

### Exemple 21 : N-{2-(7-Méthoxy-1-naphtyl)-3-[(méthylsulfonyl)amino]propyl} propanamide

On procède comme dans le Stade C de l'Exemple 1 à partir du composé obtenu dans l'Exemple 14 et en remplaçant le chorure de cyclopropanoyle par le chlorure de mésyle. Le produit du titre est recristallisé dans l'acétonitrile et obtenu sous la forme d'un solide blanc..
*Point de fusion : 140-142°C*

| *Microanalyse élémentaire :* | | | |
|---|---|---|---|
| % | *C* | *H* | *N* |
| *Calculé :* | *59,32* | *6,64* | *7,69* |
| *Trouvé :* | *59,20* | *6,80* | *7,85* |

### Exemple 22 : N-Ethyl-N'-[3-hydroxy-2-(7-méthoxy-1-naphtyl)propyl]urée

Le composé obtenu dans le Stade B de l'Exemple 1 (5,6 mmol) est mis en solution dans 40 ml de tétrahydrofurane en présence de triéthylamine (1,56 mmol), et l'isocyanated'éthyle (2,5 mmol) est ajouté. Le milieu est agité à température ambiante pendant 10 minutes, concentré sous pression réduite puis versé dans l'eau. La phase aqueuse est extraite par 2 fois 60 ml d'éther, la phase organique est lavée avec une solution d'acide chlorhydrique (1M), puis lavée à l'eau, séchée et évaporée. L'huile obtenue précipite dans un mélange éther-éther de pétrole (50/50), le précipité formé est essoré puis recristallisé dans l'acétonitrile pour conduire au composé du titre sous la forme d'un solide blanc.
*Point de fusion : 120-122°C*

| *Microanalyse élémentaire :* | | | |
|---|---|---|---|
| % | *C* | *H* | *N* |
| *Calculé :* | *67,53* | *7,33* | *9,26* |
| *Trouvé :* | *67,47* | *7,21* | *9,17* |

### Exempte 23 : N-[2-(3-Bromo-7-méthoxy-1-naphtyl)-3-hydroxypropyl]acétamide

On procède comme dans l'Exemple 1 en remplaçant au stade A le (7-méthoxy-napht-1-yl) acétonitrile par le (3-bromo-7-méthoxy-napht-1-yl)acétonitrile, et au stade C le chlorure de cyclopropanoyle par le chlorure d'acétyle. Le composé du titre est recristallisé dans l'acétonitrile et obtenu sous la forme d'un solide blanc.
*Point de fusion : 129-131°C*

### Exemple 24 : N-[2-(3-Allyl-7-méthoxy-1-naphtyl)-3-hydroxypropyl]acétamide

On procède comme dans l'Exemple 23 en remplaçant au Stade A le (7-méthoxy-napht-1-yl)acétonitrile par le (3-allyl-7-méthoxy-napht-1-yl)acétonitrile. Le composé du titre est recristallisé dans l'acétonitrile et obtenu sous la forme d'un solide blanc.
*Point de fusion : 142-144°C*

### Exemple 25 : N-[2-(3-Allyl-7-méthoxy-1-naphtyl)-3-hydroxypropyl]acétamide

On procède comme dans l'Exemple 23 en remplaçant au Stade A le (7-méthoxy-napht-1-yl)acétonitrile par le (7-méthoxy-3-vinyl-napht-1-yl)acétonitrile. Le composé du titre est recristallisé dans l'acétonitrile et obtenu sous la forme d'un solide blanc.
*Point de fusion : 109-111°C*

### Exemple 26 : N-[4-Hydroxy-2-(7-méthoxy-1-naphtyl)butyl]cyclopropanecarboxamide

### Stade A : 4-Amino-3-(7-méthoxy-1-naphtyl)-1-butanol, chlorhydrate

Le chlorure d'aluminium (80 mmol), solubilisé dans 200 ml d'éther anhydre, est ajouté à une suspension d'aluminohydrure de lithium (80 mmol) à 0°C dans 300 ml d'éther anhydre. Après 10 minutes d'agitation le composé obtenu au Stade B de l'Exemple 7 (20 mmol) en solution dans 200 ml d'éther anhydre est ajouté. Après 30 minutes, le milieu est hydrolysé à froid et avec précaution avec une solution de soude (250 mmol). Le précipité minéral formé est ensuite filtré et abondamment lavé à l'éther. Le résidu obtenu après évaporation est repris par l'eau, et la phase aqueuse est extraite au dichlorométhane. La phase organique est ensuite lavée à l'eau, séchée, décolorée, puis traitée par l'acide chlorhydrique gazeux et évaporée. L'huile obtenue précipite dans l'acétate d'éthyle et le précipité formé est essoré puis recristallisé pour conduire au produit du titre sous la forme d'un solide blanc.
*Point de fusion : 164-166°C*

### Stade B : N-[4-Hydroxy-2-(7-méthoxy-1-naphtyl)butyl]cyclopropane carboxamide

Le composé obtenu au Stade A (20 mmol) est dissous dans un mélange eau/acétate d'éthyle (25 ml/75 ml) refroidi à 0°C. Le carbonate de potassium (60 mmol) est ajouté puis le chlorure de l'acide cyclopropane carboxylique (26 mmol) est additionné goutte à goutte au milieu réactionnel. L'ensemble est mis sous agitation vigoureuse pendant 30 minutes à température ambiante. Les deux phases sont séparées et la phase organique est lavée par une solution aqueuse d'acide chlorhydrique 0,1M puis par de l'eau. Après séchage sur sulfate de magnésium, la phase organique est évaporée sous pression réduite. Le résidu obtenu est recristallisé pour conduire au produit du titre sous la forme d'un solide blanc.
*Point de fusion : 158-160°C*

### Exemple 27 : N-[4-Hydroxy-2-(7-méthoxy-1-naphtyl)butyl]propanamide

On procède comme dans l'Exemple 26 en remplaçant au Stade B le chlorure de l'acide cyclopropane carboxylique par le chlorure de l'acide propionique. Le composé du titre est obtenu sous la forme d'un solide blanc.
*Point de fusion : 123-125°C*

### Exemple 28 : 2-Fluoro-N-[4-hydroxy-2-(7-méthoxy-1-naphtyl)butyl]acétamide

On procède comme dans l'Exemple 26 en remplaçant au Stade B le chlorure de l'acide cyclopropane carboxylique par le chlorure de l'acide fluoroacétique.
*Point de fusion : 96-98°C*

### Exemple 29 : N-[4-Hydroxy-2-(7-méthoxy-1-naphtyl)butyl]cyclobutanecarboxamide

On procède comme dans l'Exemple 26 en remplaçant au Stade B le chlorure de l'acide cyclopropane carboxylique par le chlorure de l'acide cyclobutane carboxylique. Le composé du titre est obtenu sous la forme d'un solide blanc.
*Point de fusion : 113-115°C*

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Test de la nage forcée

Les composés de l'invention sont testés dans un modèle comportemental, le test de la nage forcée.

L'appareil est constitué d'un cylindre en plexiglas rempli d'eau. Les animaux sont testés individuellement pendant une session de 6 minutes. Au début de chaque test, l'animal est placé au centre du cylindre. Le temps d'immobilisation est enregistré. Chaque animal est jugé immobile quand il cesse de se débattre, et reste à la surface de l'eau, immobile, ne faisant seulement que les mouvements lui permettant de maintenir la tête hors de l'eau.

Après administration 40 minutes avant le début du test, les composés de l'invention diminuent de façon significative la durée d'immobilisation attestant de leur activité antidépressive.

### EXEMPLE C : Etude de liaison aux récepteurs MT₁ et MT₂ de la mélatonine

Les expériences de liaison aux récepteurs MT₁ ou MT₂ sont réalisées en utilisant la 2-[¹²⁵I]-iodomélatonine comme radioligand de référence. La radioactivité retenue est déterminée à l'aide d'un compteur à scintillation liquide.
Des expériences de liaison compétitive sont ensuite réalisées en triple, avec les différents composés à tester. Une gamme de concentrations différentes est testée pour chaque composé. Les résultats permettent de déterminer les affinités de liaison des composés testés (Kᵢ).

Ainsi, les valeurs de Kᵢ trouvées pour les composés de l'invention attestent d'une liaison pour l'un ou l'autre des sites de liaisons mélatoninergiques, ces valeurs étant ≤ 10 µM.

A titre d'exemple, le composé de l'Exemple 6 présente un Kᵢ (MT₁) de 4,9 nM et un Kᵢ (MT₂) de 8,9 nM.

### EXEMPLE D : Etude de la liaison aux récepteurs sérotoninergiques 5-HT_{2C}

L'affinité des composés pour le récepteur humain 5-HT_{2C} est évaluée sur des préparations membranaires de cellules CHO exprimant de façon stable ce récepteur.
L'incubation est réalisée dans du tampon TRIS 50 mM, pH 7,4 contenant du MgCl₂ 10 mM et de la BSA 0,1%, en présence de [³H]Mésulergine (1 nM) et de 25 fmol/ml de récepteur. La liaison non-spécifique est déterminée en présence de miansérine 10µM.
La réaction est stoppée par l'ajout de tampon TRIS 50 mM, pH 7,4 suivi d'une étape de filtration et de 3 rinçages successifs : la radioactivité liée aux membranes restant sur les filtres (GF/B prétraités au PEI 0.1%) est comptée en scintillation liquide.

Les résultats obtenus montrent que les composés de l'invention sont affins pour le récepteur 5-HT_{2C} avec des Kᵢ < 100µM.

A titre d'exemple, le composé de l'Exemple 6 présente un Kᵢ (5-HT_{2C}) de 26 µM.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE F : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.
Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE G : Composition pharmaceutique: Comprimés

| 1000 comprimés dosés à 5 mg de *N*-[3-méthoxy-2-(7-méthoxy-1-naphtyl)propyl]acétamide | |
|---|---|
| (Exemple 6) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 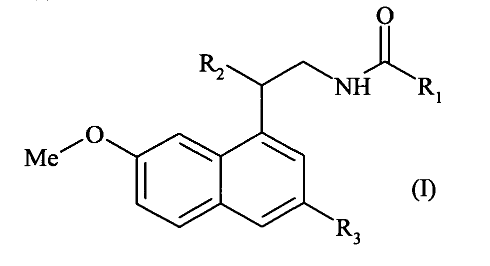 dans laquelle :
◆ R₁ représente un groupement R₄ ou NHR₄, dans lesquels R₄ représente groupement alkyle (C₁-C₆) linéaire ou ramifié, alkényle (C₁-C₆) linéaire ou ramifié, halogénoalkyle (C₁-C₆) linéaire ou ramifié, polyhalogénoalkyle (C₁-C₆) linéaire ou ramifié, cycloalkyle (C₃-C₈), cycloalkyl(C₃-Cg)alkyle (C₁-C₆) dont la partie alkyle peut être linéaire ou ramifiée, aryle, arylalkyle (C₁-C₆) dont la partie alkyle peut être linéaire ou ramifiée, hétéroaryle ou hétéroarylalkyle (C₁-C₆) dont la partie alkyle peut être linéaire ou ramifiée,
◆ R₂ représente un groupement alkyle (C₁-C₆) linéaire ou ramifié substitué par un groupement alkoxy (C₁-C₆) linéaire ou ramifié, OH, OSO₂Me, N₃, NRR', NHCOR" ou par un groupement NHSO₂R",
dans lesquels R et R', identiques ou différents, représentent chacun un atome d'hydrogène ou un groupement alkyle (C₁-C₆) linéaire ou ramifié, cycloalkyle (C₃-C₈), aryle ou arylalkyle (C₁-C₆) dont la partie alkyle peut être linéaire ou ramifiée, ou R et R' forment ensemble avec l'atome d'azote qui les porte un cycle comportant 5 ou 6 chaînons et pouvant contenir un autre hétéroatome choisi parmi azote, oxygène et soufre,
et R" représente un groupement alkyle (C₁-C₆) linéaire ou ramifié, cycloalkyle (C₃-C₈), aryle ou arylalkyle (C₁-C₆) dont la partie alkyle peut être linéaire ou ramifiée,
◆ R₃ représente un atome d'hydrogène ou d'halogène, ou un groupement alkyle (C₁-C₆) linéaire ou ramifié ou alkényle (C₁-C₆) linéaire ou ramifié,
étant entendu que :
- lorsque R₁ représente un groupement méthyle et R₂ représente un groupement hydroxyméthyle, alors R₃ ne peut représenter un atome d'hydrogène,
- par "aryle", on entend un groupement phényle, naphtyle ou biphényle,
- par "hétéroaryle", on entend tout groupement aromatique mono ou bicyclique contenant 1 à 3 hétéroatomes choisis parmi oxygène, soufre et azote,
les groupements aryle et hétéroaryle ainsi définis pouvant être substitués par 1 à 3 groupements choisis parmi alkyle (C₁-C₆) linéaire ou ramifié, alkoxy (C₁-C₆) linéaire ou ramifié, hydroxy, carboxy, formyle, nitro, cyano, haloalkyle (C₁-C₆) linéaire ou ramifié, polyhalogénoalkyle (C₁-C₆) linéaire ou ramifié, alkyloxycarbonyle, ou atomes d'halogène,
leurs énantiomères et diastéréoisomères, ainsi que leurs sels d'addition à un acide ou à une base pharmaceutiquement acceptable.

2. Composés de formule (I) selon la revendication 1 pour lesquels R₁ représente un groupement alkyle, leurs énantiomères et diastéréoisomères ainsi que leurs sels d'addition à un acide ou à une base pharmaceutiquement acceptable.

3. Composés de formule (I) selon la revendication 1 pour lesquels R₂ représente un groupement alkyle substitué par un groupement hydroxy, leurs énantiomères et diastéréoisomères, ainsi que leurs sels d'addition à un acide ou à une base pharmaceutiquement acceptable.

4. Composés de formule (I) selon la revendication 1 pour lesquels R₂ représente un groupement alkyle substitué par un alkoxy, leurs énantiomères et diastéréoisomères, ainsi que leurs sels d'addition à un acide ou à une base pharmaceutiquement acceptable.

5. Composés de formule (I) selon la revendication 1 pour lesquels R₃ représente un atome d'hydrogène, leurs énantiomères et diastéréoisomères, ainsi que leurs sels d'addition à un acide ou à une base pharmaceutiquement acceptable.

6. Composé de formule (I) selon la revendication 1 qui est le *N*-[3-méthoxy-2-(7-méthoxy-1-naphtyl)propyl]acétamide, ses énantiomères ainsi que ses sels d'addition à un acide ou à une base pharmaceutiquement acceptable.

7. Composé de formule (I) selon la revendication 1 qui est le *N*-[4-hydroxy-2-(7-méthoxy-1-naphtyl) butyl]acétamide, ses énantiomères ainsi que ses sels d'addition à un acide ou à une base pharmaceutiquement acceptable.

8. Composé de formule (I) selon la revendication 1 qui est le *N*-[4-hydroxy-2-(7-méthoxy-1-naphtyl) butyl]propanamide, ses énantiomères ainsi que ses sels d'addition à un acide ou à une base pharmaceutiquement acceptable.

9. Procédé de préparation des composés de formule (I) selon la revendication 1 **caractérisé en ce que** l'on utilise comme produit de départ le composé de formule (II) : 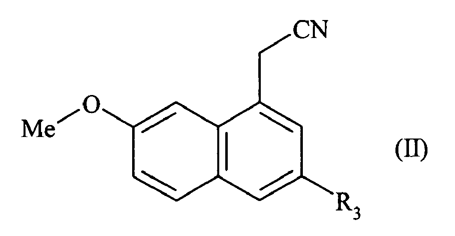 dans laquelle R₃ est tel que défini dans la revendication 1,
que l'on soumet en milieu basique à l'action du carbonate de diméthyle pour conduire au composé de formule (III) : 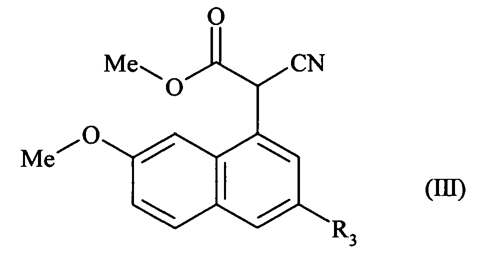 dans laquelle R₃ est tel que défini précédemment, sur lequel on condense éventuellement un composé de formule Hal-(CH₂)ₙ-COOMe dans laquelle Hal représente un atome d'halogène et n vaut 1 à 6 pour conduire au composé de formule (IV): 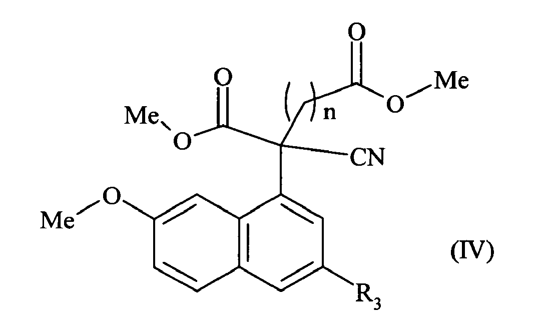 dans laquelle R₃ et n sont tels que définis précédemment,
que l'on soumet à l'action du bromure de lithium pour conduire au composé de formule (V) : 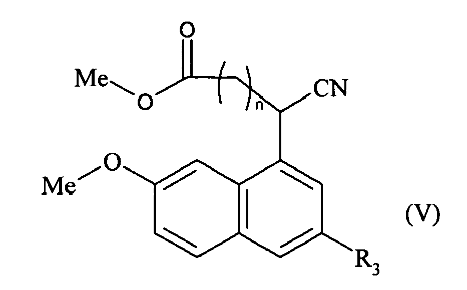 dans laquelle R₃ et n sont tels que définis précédemment, l'ensemble des composés de formule (III) et (V) formant le composé de formule (VI) : 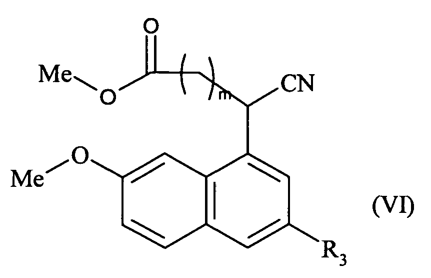 dans laquelle R₃ est tel que défini précédemment et m vaut 0, 1, 2, 3, 4, 5 ou 6,
que l'on soumet à une réduction en présence d'un hydrure pour conduire au composé de formule (VII) : 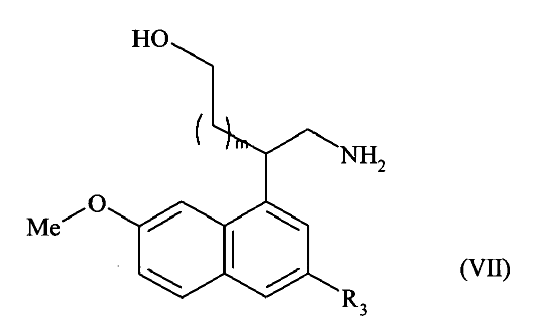 dans laquelle R₃ et m sont tels que définis précédemment,
sur lequel on condense un composé de formule R₁C(O)Cl pour conduire au composé de formule (I/a), cas particulier des composés de formule (I) : 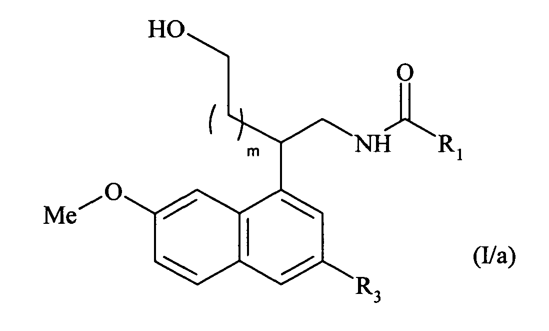 dans laquelle R₃, m et R₁ sont tels que définis précédemment, qui est éventuellement :
- soit soumis à l'action d'un halogénure d'alkyle en milieu basique pour conduire au composé de formule (I/b), cas particulier des composés de formule (I) : 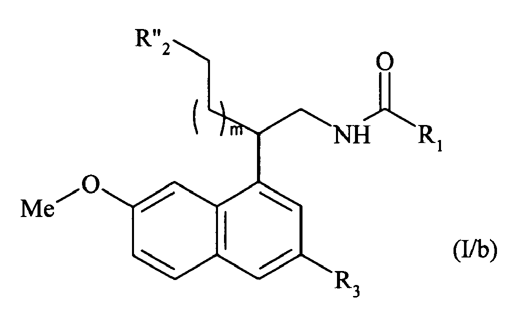 dans laquelle R₃, m et R₁ sont tels que définis précédemment et R"₂ représente un groupement alkoxy (C₁-C₆) linéaire ou ramifié,
- soit sur lequel on condense le chlorure de mésylate en milieu basique pour conduire au composé de formule (I/c), cas particulier des composés de formule (I) : 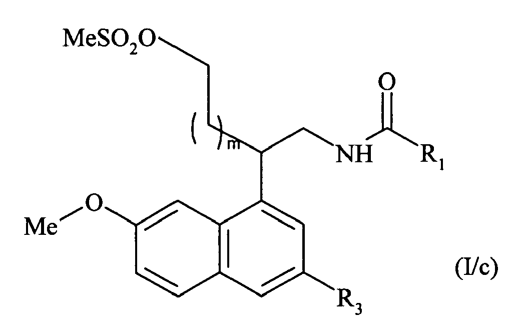 dans laquelle R₃, m et R₁ sont tels que définis précédemment, sur lequel on condense éventuellement :
□ soit une amine de formule HNRR' dans laquelle R et R' sont tels que définis dans la revendication 1 pour conduire au composé de formule (I/d), cas particulier des composés de formule (I) : 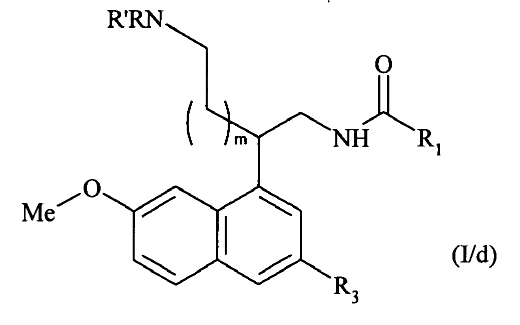 dans laquelle R₃, m, R, R' et R₁ sont tels que définis précédemment,
□ soit un azidure pour conduire au composé de formule (I/e), cas particulier des composés de formule (I), 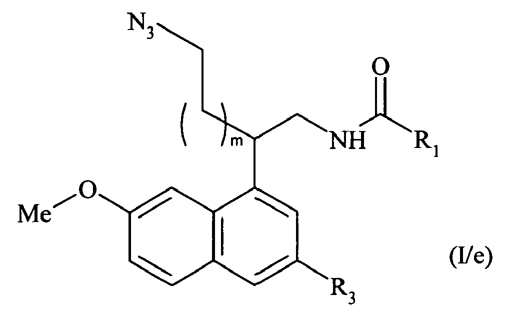 dans laquelle R₃, m et R₁ sont tels que définis précédemment,
que l'on soumet éventuellement à une réduction en présence de charbon palladié, suivi éventuellement de la mono ou bis condensation d'un composé de formule R-Hal dans laquelle R est tel que défini dans la revendication 1, pour conduire au composé de formule (I/d) tel que défini précédemment pour lequel R et R' ne forment pas ensemble avec l'atome d'azote qui les porte un groupement cyclique,
composé de formule (I/d) qui, lorsque R et R' représentent simultanément un atome d'hydrogène est éventuellement soumis à l'action d'un composé de formule R"C(O)Cl ou R"SO₂Cl dans lesquelles R" est tel que défini précédemment, pour conduire au composé de formule (I/f), cas particulier des composés de formule (I) : 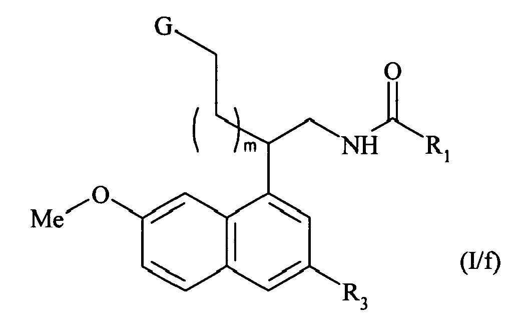 dans laquelle R₃, m et R₁ sont tels que définis précédemment, et G représente un groupement NHCOR" ou NHSO₂R", dans lesquels R" est tel que défini précédemment,
les composés de formule (I/a) à (I/f) formant l'ensemble des composés de formule (I) qui peuvent être purifiés selon une technique classique de séparation, que l'on transforme, si on le souhaite, en leurs sels d'addition à un acide ou à une base pharmaceutiquement acceptable et dont on sépare éventuellement les isomères selon une technique classique de séparation.

10. Compositions pharmaceutiques contenant au moins un composé de formule (I) selon l'une quelconque des revendications 1 à 8 ou un de leurs sels d'addition avec un acide ou une base pharmaceutiquement acceptable en combinaison avec un ou plusieurs excipients pharmaceutiquement acceptables.

11. Compositions pharmaceutiques selon la revendication 10 utiles pour la fabrication de médicaments pour traiter les troubles du système mélatoninergique.

12. Compositions pharmaceutiques selon la revendication 10 utiles pour la fabrication de médicaments pour le traitement des troubles du sommeil, du stress, de l'anxiété, de la dépression majeure ou des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, des troubles de la circulation cérébrale, ainsi que dans les dysfonctionnements sexuels, en tant qu'inhibiteurs de l'ovulation, d'immunomodulateurs et dans le traitement des cancers.
